# FASCICULE DE BREVET EUROPEEN

(11) **EP 2 861 338 B1**
(45) Date de publication et mention de la délivrance du brevet: **23.01.2019**
(21) Numéro de dépôt: 13728393.3
(22) Date de dépôt: 13.06.2013
(51) Int. Cl.: B01J 13/04, A61K 9/50

(54) **PROCEDE POUR L'OBTENTION D'UNE STRUCTURE DE CAPSULE NANOMETRIQUE**
VERFAHREN ZUR HERSTELLUNG EINER NANOKAPSELSTRUKTUR
METHOD FOR OBTAINING A NANO-CAPSULE STRUCTURE

(30) Priorité: 14.06.2012 FR 1255561
(43) Date de publication de la demande: 22.04.2015
(73) Titulaire: Université de Lorraine, 54000 Nancy (FR); Centre National de la Recherche Scientifique (C.N.R.S.), 75794 Paris Cedex 16 (FR)
(72) Inventeur: MC MURTRY, Stefan, F-54520 Laxou (FR); ELMAZRIA, Omar, F-54280 Seichamps (FR)
(74) Mandataire: Rhein, Alain
(86) Numéro de dépôt international: PCT/EP2013/062221
(87) Numéro de publication internationale: WO 2013/186292

(56) Documents cités:
- EP-A1- 0 512 693
- WO-A1-95/24472
- WO-A1-2011/102809
- WO-A2-01/41736
- US-A- 5 893 974
- US-A1- 2003 029 558
- US-A1- 2004 113 543
- US-A1- 2009 242 382
- DATABASE WPI Week 201157 Thomson Scientific, London, GB; AN 2011-K50337 XP002726674, & JP 2011 158665 A (SONY CORP) 18 août 2011 (2011-08-18)
- DATABASE WPI Week 200902 Thomson Scientific, London, GB; AN 2009-A40396 XP002726675, -& KR 100 834 247 B1 (DONGBU ELECTRONICS CO LTD) 30 mai 2008 (2008-05-30)

## Description

La présente invention concerne le domaine des structures de capsule nanométrique.

La présente invention pourra trouver son application dans de très nombreux domaines en raison des propriétés particulières de telles structures. A titre d'exemple, l'invention trouvera une application dans le domaine de la production d'énergie, ou encore dans le domaine de l'encapsulation de composés, que ces derniers soient solides, liquides ou gazeux, y compris le vide, c'est-à-dire à une pression inférieure à la pression atmosphérique.

L'invention concerne plus particulièrement un procédé pour l'obtention de structures de capsule nanométrique aptes à permettre l'encapsulation d'un composé. Ainsi, ces structures peuvent également être appelées nanoréservoirs, et permettre une encapsulation physique de composés divers sous les trois états usuels de la matière : solides, liquides ou gazeux. Il est également possible d'encapsuler du vide.

A l'heure actuelle, l'encapsulation chimique de composés est bien connue. Les procédés d'encapsulation chimique consistent à enrober une molécule dans le but de protéger cette dernière. L'objectif principal poursuivi par de tels procédés est de permettre une libération de la molécule, encapsulée par voie chimique, dans une situation particulière. Par exemple, on peut souhaiter obtenir une dissolution progressive de l'enrobage dans le tube digestif de manière à libérer le principe actif qu'il renferme.

On connait ainsi, par le document de l'état de la technique WO 2009/039458, l'encapsulation de microstructures ou de nanostructures avec des gels polymériques, plus particulièrement des microgels. De telles structures peuvent notamment permettre l'encapsulation de virus, ces derniers pouvant servir de matrice à la synthèse de nanostructures ou de microstructures.

On connait encore, par le document WO 2007/003054, un procédé relatif à l'immobilisation de bio-molécules sur des polymères, d'origine naturelle ou obtenus par synthèse chimique, ces derniers pouvant être fonctionnalisés sous forme de nanoparticules pour permettre une liaison de la biomolécule à la nanoparticule. La liaison entre biomolécules et particules de polymères, par exemple les nanoparticules, se fait selon une réaction chimique qui est une cycloaddition. Des molécules ou des agents thérapeutiques peuvent être encapsulés à l'intérieur des particules, de manière à permettre leur délivrance notamment par voie intraveineuse.

Cependant, l'encapsulation par voie chimique présente plusieurs inconvénients. D'une part, chaque type de molécule nécessite une encapsulation particulière ; en effet, certaines molécules sont plus fragiles que d'autres et sont susceptibles d'être détruites par la solution d'enrobage. En conséquence, toutes les molécules ne peuvent pas être enrobées par la voie de l'encapsulation chimique. D'autre part, ce type d'encapsulation présente également des limitations sur l'état du composé à encapsuler et sur la quantité. En particulier, les composés gazeux et liquides ne peuvent pas être encapsulés selon la voie chimique. De plus, les agrégats moléculaires ne peuvent être encapsulés que dans le cas où la solution d'enrobage ne les dissout pas. Enfin, le fait de positionner des molécules encapsulées par voie chimique sur un substrat est particulièrement laborieux, ce qui restreint les champs d'application possibles.

Les procédés consistant à enrober un composé par voie chimique présentent certaines limitations et ne donnent pas entièrement satisfaction.

On connait encore, du document de brevet WO 2011/102809, un procédé d'encapsulation par voie chimique qui tente de répondre aux inconvénients précités, en particulier en permettant une encapsulation de composés liquides, solides, ou gazeux.

Ce document concerne un procédé pour l'obtention d'une pluralité de micro-chambres. Dans une première étape de ce procédé, un réseau de micro-puits est imprimé par nanoimpression ou lithographie électronique sur un substrat en polymère. Dans une seconde étape il est appliqué une première couche, également en polymère, sur ce substrat structuré.

Cette première couche consiste plus particulièrement en une succession de plusieurs bicouches, au moins 10, comportant chacune deux couches chargées de manière différente. En d'autres termes, chaque bicouche comporte une couche de polymère anionique et une couche de polymère cationique.

Un composé quelconque est ensuite chargé dans les micro-puits, puis une couche de scellage est appliquée.

Le dépôt couche par couche (ou « layer by layer ») sur le substrat est réalisé par trempage du substrat dans les différentes solutions de polymère ou en mettant en oeuvre une technique de pulvérisation. Une telle technique, appelée « spray coating » en anglais, est adaptée au dépôt de polymères en phase liquide et consiste en une propulsion, par un flux d'air, dudit polymère dissout dans un solvant.

Le procédé décrit dans ce document présente cependant encore un certain nombre d'inconvénients.

En particulier, les techniques de dépôt de la première couche sont imprécises et les matériaux polymères utilisés ne sont pas adaptés à l'obtention d'une couche encapsulante d'épaisseur parfaitement contrôlée tout en étant extrêmement mince et particulièrement résistante ; de ce fait, le procédé permet uniquement d'obtenir une matrice de micro-chambres, et non pas des structures isolées, et la matrice ainsi obtenue ne permet pas de libérer chacune des micro-chambres indépendamment les unes des autres sans entrainer la déstructuration de cette couche de polymère. On observera encore que, étant donné que le substrat ainsi que la première couche sont en matériau polymère, se pose le problème de la séparation des micro-chambres du substrat. Dans tous les cas, cela impose le choix de polymères particuliers, d'une part pour le substrat et d'autre part pour ladite première couche.

De plus, il est difficile d'obtenir des structures de très petite taille, de l'ordre du nanomètre, par la mise en oeuvre des étapes du procédé décrit dans ce document. En effet, le dépôt de la première couche par trempage en solution, ou par une autre technique citée, ne permet pas une pénétration optimale de ladite solution dans une cavité qui présenterait des dimensions nanométriques, du fait des forces de capillarité et de tension existant entre la première couche et le substrat.

Or, l'obtention de structures individualisées et de taille nanométrique, présente un intérêt particulier dans de nombreux domaines d'application, et notamment dans le milieu médical.

Enfin, le procédé décrit dans ce document est fastidieux et long à mettre en oeuvre. En effet, outre la pluralité de trempages dans diverses solutions de polymères nécessaire à l'obtention du produit, il convient de procéder à une sonication du substrat pour éliminer les bulles d'air susceptibles d'entraîner la formation d'une couche discontinue et non uniforme lors des opérations de trempage.

Les inventeurs ont alors cherché à développer une technique afin de remédier aux inconvénients des techniques chimiques d'encapsulation, et permettant l'obtention de structures de capsule nanométrique pouvant notamment aboutir à une encapsulation physique de composés.

En ce qui concerne les techniques physiques, il est notamment connu de l'état de la technique, un procédé appelé « lift off » qui permet de structurer une résine généralement déposée sur un substrat. Plus particulièrement, le lift off consiste à imprimer, dans une couche de résine déposée à la surface d'un substrat, un motif inverse par lithographie électronique ou optique. Une fine couche de métal est ensuite déposée par évaporation, de manière à essayer de recouvrir la résine restante et les parties du substrat desquelles a été éliminée ladite résine par lithographie. Pour terminer, la résine restante est éliminée, par exemple par dissolution, de manière à conserver uniquement la couche de métal se trouvant au contact du substrat.

Ainsi, en procédant à une étape de lithographie suivie d'un dépôt de métal par évaporation et d'un décollage de la résine, il est par exemple possible d'obtenir une structuration de plots d'or sur silicium.

On connait aussi, par le document de brevet WO 2007/072247 un procédé lift off particulier dans lequel un profil en casquette est formé pour permettre l'obtention d'une couche de métal présentant une forme latérale prédéfinie, à une position prédéfinie d'une surface d'un matériau semi-conducteur. De la même manière que la technique citée précédemment, celle-ci permet uniquement la structuration de plots à la surface d'une couche de substrat.

Les techniques existantes actuellement dans le domaine de la structuration de surfaces permettent ainsi uniquement l'obtention de structures monolithiques, tels que des plots, et non pas des structures creuses, et comportant des parois fines, destinées à permettre l'encapsulation de composés, qu'ils soient liquides, solides, ou gazeux.

De plus, la technique de dépôt d'un métal par évaporation n'est pas entièrement satisfaisante. Il existe en effet des difficultés à obtenir une couche de métal continue, ou isotrope, à la surface d'une résine. Par ailleurs, les techniques de structuration de résine ne sont pas totalement adaptées, et conduisent à un percement de la résine, entrainant des difficultés supplémentaires dans la fabrication de structures fermées.

L'invention offre la possibilité de pallier les nombreux inconvénients de l'état de la technique en proposant un procédé original permettant d'obtenir des structures de capsule nanométrique présentant une forme quelconque, comportant une couche mince et continue d'au moins un matériau formant lesdites structures qui, selon le cas, facilite la séparation en capsules nanométriques individuelles.

Par ailleurs, ces capsules nanométriques sont susceptibles, d'encapsuler un composé solide, liquide ou gazeux, y compris du vide.

A cet effet, la présente invention concerne un procédé, pour l'obtention d'une structure de capsule nanométrique, comportant au moins une capsule de taille nanométrique, ledit procédé comportant les étapes suivantes :
- on dépose au moins une couche de résine sur une couche de substrat;
- on structure ladite couche de résine, par lithographie électronique à faible tension, ou par lithographie optique ou par nanoimpression de manière à obtenir au moins une cavité dans l'épaisseur de ladite couche de résine, ladite cavité présentant une profondeur p inférieure à l'épaisseur E de ladite couche de résine ;
- on effectue un dépôt isotrope d'au moins une couche d'un matériau encapsulant par pulvérisation cathodique sous vide dudit matériau;
- on obture la/les cavité(s) avec un matériau d'obturation ;
- on dissout la couche de résine par trempage dans un solvant adapté.

De préférence, le procédé selon la présente invention permet d'obtenir une structure de capsule nanométrique apte à permettre l'encapsulation d'un composé.

On entend par dépôt isotrope un dépôt d'une fine couche, préférentiellement constituée par un matériau métallique, semi-conducteur, isolant ou piézoélectrique, et qui est continue sur l'ensemble de la résine. En particulier, le procédé selon la présente invention permet de déposer une fine couche de matériau métallique, semi-conducteur piézoélectrique ou isolant sur l'ensemble de la couche de résine et dans les cavités formées dans celle-ci, les parois latérales desdites cavités étant également recouvertes par le matériau déposé, grâce à l'étape de dépôt par pulvérisation cathodique sous vide.

Un dépôt isotrope s'oppose à un dépôt anisotrope, obtenu par la mise en oeuvre des techniques actuellement utilisées, pour lequel il persiste des zones de non dépôt, non recouvertes par le matériau.

Selon d'autres caractéristiques de l'invention :
- suite à l'étape de dépôt isotrope d'au moins une couche d'un matériau encapsulant par pulvérisation cathodique sous vide, l'on remplit la/les cavité(s) d'un composé quelconque, ledit composé pouvant être liquide, solide ou gazeux, y compris du vide ;
- on structure la couche de résine par lithographie électronique à faible tension, cette dernière étant comprise entre 1 et 15kV ; préalablement à la structuration de la couche de résine par lithographie électronique, on peut réaliser un test d'approche itérative pour déterminer la dose d'électrons à envoyer sur la couche de résine, de manière à éviter un percement de ladite couche de résine jusqu'à la couche de substrat ;
- on dépose par enduction centrifuge une couche de résine présentant une épaisseur E comprise entre 300 nm et 2pm et on structure ladite couche de résine de manière à obtenir une cavité présentant une profondeur p comprise entre 20nm et 1µm, de préférence entre 50 et 500nm, de préférence entre 50 et 300nm, ladite profondeur p étant inférieure à ladite épaisseur E ;
- on effectue le dépôt isotrope par pulvérisation cathodique sous vide de matériau encapsulant à une puissance comprise entre 80 et 120 Watts, préférentiellement égale à 100W ;
- on effectue toutes les étapes dudit procédé à une température contrôlée comprise entre 15°C et 30°C, de préférence entre 18 et 25°C, de préférence égale à 20°C ;
- on pulvérise au moins une couche de matériau encapsulant dont l'épaisseur e est comprise entre 10 et 200nm ;
- on dépose une couche de résine constituée de poly(méthyle méthacrylate) et/ou de poly(méthyle méthacrylate acide méthacrylique) ;
- le substrat est constitué par un matériau souple ou rigide choisi parmi le silicium, l'oxyde de zinc, le nitrure d'aluminium, l'oxyde de silicium, l'oxyde d'aluminium, les polymères, les matériaux plastiques ;
- on dépose un matériau encapsulant pulvérisable choisi parmi les matériaux métalliques, les matériaux semi-conducteurs, les matériaux piézoélectriques et les matériaux isolants ;
- on obture la/les cavité(s) avec un matériau d'obturation choisi parmi les polymères, les métaux, les résines époxydes et les résines biocompatibles, ledit matériau d'obturation étant apte à résister au solvant utilisé dans l'étape de dissolution de la résine ;
- préalablement à l'étape de dissolution de la couche de résine, on transfère la structure de capsule nanométrique sur un second substrat en appliquant une résine de collage à la surface du matériau d'obturation puis on dépose un second substrat sur ladite résine de collage ; on peut également transférer la structure de capsule nanométrique sur un second substrat en déposant ledit second substrat à la surface du matériau d'obturation, ce dernier constituant alors une résine de collage pour le transfert de la structure de capsule nanométrique sur un second substrat ;
- suite à l'étape de transfert de la structure de capsule nanométrique sur un second substrat, l'on effectue une pulvérisation cathodique sous vide d'au moins une seconde couche d'un matériau métallique ou isolant ou semi-conducteur ou piézoélectrique sur la couche de matériau encapsulant ;
- suite à l'obtention de la structure de capsule nanométrique par obturation de la/des cavité(s) avec un matériau d'obturation, le procédé comporte encore les étapes suivantes :
   - on structure, par alignement avec la première structuration de la couche de résine, la couche de matériau d'obturation par lithographie électronique ou par lithographie optique ou par nanoimpression, de manière à éliminer ledit matériau excepté au niveau de la/des capsule(s) nanométrique(s) qui restent obturées ;
   - on élimine la couche de matériau encapsulant par gravure chimique ou par trempage ou par gravure ionique réactive ou par gravure par faisceau d'ions;
   - après dissolution de la couche de résine par trempage dans un solvant adapté, on libère ladite/lesdites capsule(s) nanométrique(s) en solution.

La présente divulgation concerne également une structure de capsule nanométrique comportant au moins une capsule de taille nanométrique apte à permettre l'encapsulation d'un composé et formée par un contenant d'une profondeur p donnée et présentant une ouverture obturée par un opercule formé par le matériau d'obturation, le contenant comprenant une enveloppe comportant au moins une couche d'un matériau encapsulant dont l'épaisseur e respecte le rapport suivant : p/e supérieur à 2, de préférence supérieur à 6 et de préférence supérieur à 10.

La divulgation est encore relative à l'utilisation d'au moins une structure de capsule nanométrique pour l'encapsulation d'un composé quelconque.

Préférentiellement, une structure de capsule nanométrique selon l'invention peut être utilisée pour la libération du composé sur commande, par application d'un moyen permettant la destruction de l'enveloppe de la/des capsules nanométriques de ladite structure.

La divulgation concerne encore l'utilisation de la structure de capsule nanométrique pour générer de l'électricité ou pour fabriquer un nano-résonateur à ondes acoustiques ou pour fabriquer une photodiode ou pour fabriquer une source optique omnidirectionnelle.

La présente invention comporte de nombreux avantages. D'une part, le procédé selon l'invention est aisé à mettre en oeuvre, et ne nécessite pas un trop grand nombre d'étapes. D'autre part, il permet l'obtention de structures de capsule nanométrique comportant au moins une capsule nanométrique refermée et creuse à l'intérieur, de manière à permettre le remplissage éventuel de la capsule avec un composé quelconque.

En particulier, un tel composé peut consister en un principe actif de type médicament, et la présente invention permet d'encapsuler un tel principe actif de manière à favoriser une libération contrôlée de ce dernier.

Le procédé selon la présente invention, et la structure de capsule nanométrique qui en découle, peuvent permettre l'incorporation d'un composé de toute nature physique (solide, liquide ou gazeux, y compris le vide), contrairement aux structures chimiques existantes qui ne permettent notamment pas une encapsulation de molécules à l'état gazeux ou à l'état liquide.

Enfin, le présent procédé permet, de façon inédite, l'obtention d'une structure de capsule nanométrique présentant une couche mince et résistante d'un matériau encapsulant, ladite structure pouvant permettre l'encapsulation d'un composé et la libération des capsules nanométriques individuellement. De plus, ces structures de capsule nanométrique sont susceptibles d'être utilisées dans des domaines d'application variés, par exemple en médecine ou dans le domaine de la production d'énergie.

D'autres caractéristiques et avantages de l'invention ressortiront de la description détaillée qui va suivre des modes de réalisation non limitatifs de l'invention, en référence aux figures annexées dans lesquelles :
- la figure 1 représente schématiquement les étapes du procédé connu d'encapsulation chimique ;
- les figures 2A à 2C illustrent de façon schématique les étapes du procédé selon la présente invention pour l'obtention d'une structure de capsule nanométrique ;
- les figures 3A à 3D représentent un mode de réalisation particulier pour la libération de capsules nanométriques;
- la figure 4 illustre schématiquement un mode de réalisation d'une des étapes du procédé selon l'invention, dans laquelle la structure de capsule nanométrique est transférée par collage sur un second substrat ;

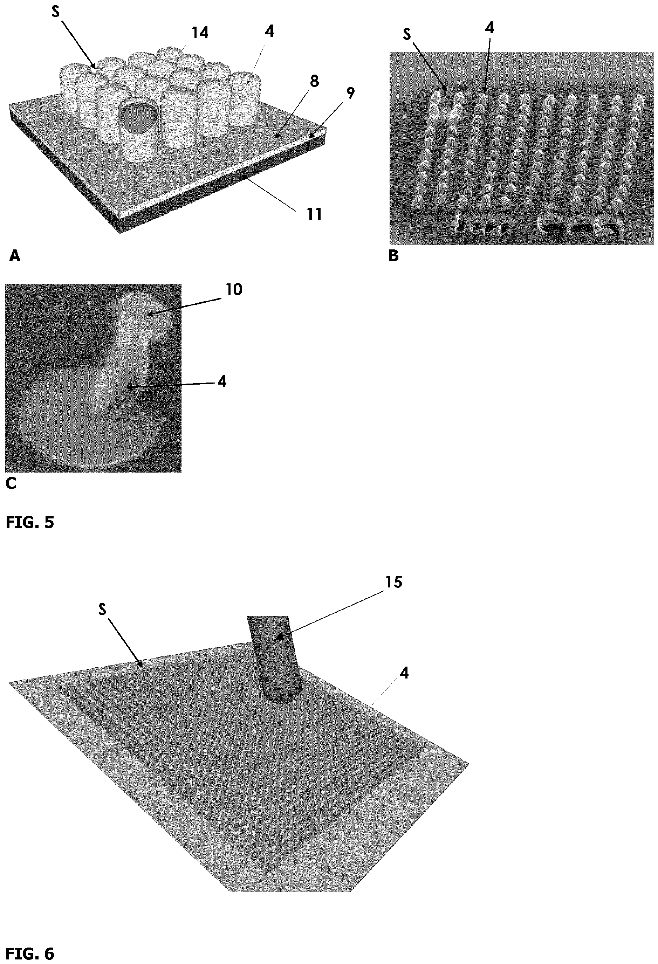

- la figure 5A représente schématiquement une structure de capsule nanométrique apte à permettre l'encapsulation d'un composé ; la figure 5B correspond à une vue de la structure selon la divulgation comportant une pluralité de capsules nanométriques ; lesdites capsules nanométriques présentent un diamètre de 300nm et sont observées au microscope électronique à balayage avec un grandissement de 13000 ; la figure 5C correspond à une capsule nanométrique individuelle, obturée avec un matériau d'obturation de type résine SU8, et vue au microscope électronique à balayage avec un grandissement de 40000 ;
- la figure 6 illustre schématiquement une des applications possibles de la structure de capsule nanométrique selon la divulgation ;
- la figure 7 représente schématiquement une seconde application possible de la structure de capsule nanométrique ;
- la figure 8 représente schématiquement une troisième application possible de capsules nanométriques individuelles ;
- les figures 9A et 9B représentent respectivement une photographie au microscope électronique à balayage (grandissement de 8000) et une représentation schématique d'une quatrième application possible de la structure de capsule nanométrique obtenue par la mise en oeuvre du procédé selon l'invention ;
- les figures 10A et 10B représentent respectivement une photographie au microscope électronique à balayage (grandissement de 7500) et une représentation schématique d'une cinquième application possible de la structure de capsule nanométrique obtenue par la mise en oeuvre du procédé selon l'invention ;
- la figure 11 est une représentation schématique d'une sixième application possible de la structure de capsule nanométrique obtenue par la mise en oeuvre du présent procédé.

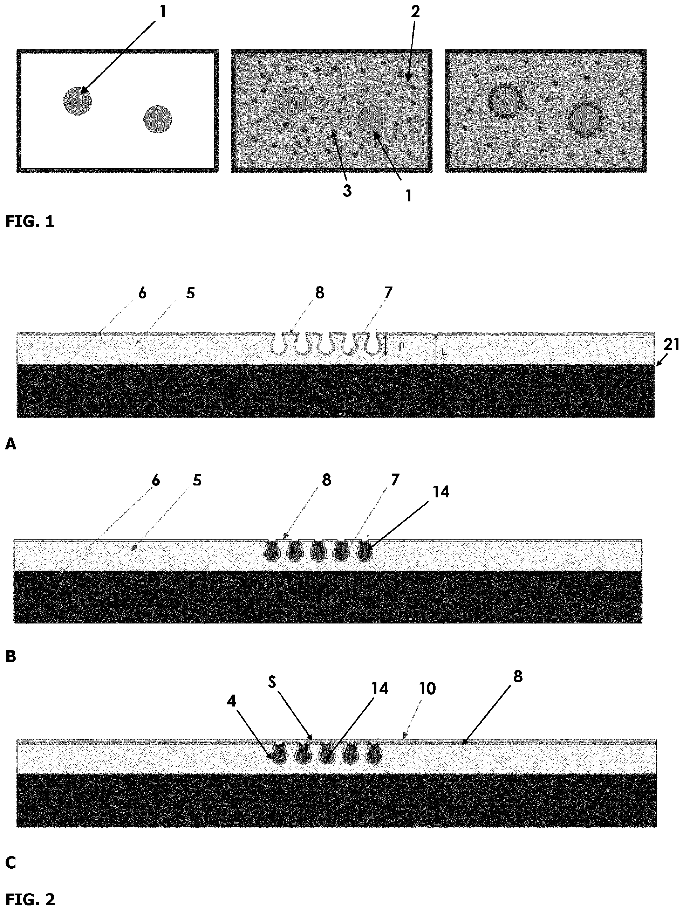

La figure 1 illustre schématiquement une des techniques utilisées dans l'état de l'art pour encapsuler de façon chimique des molécules, par exemple des principes actifs médicamenteux. Les molécules 1 qui doivent être encapsulées peuvent être mises en présence d'une solution d'enrobage 2 comportant un matériau d'enrobage 3, tel qu'un polymère, présentant une affinité pour la molécule 1 qui doit être encapsulée.

Comme évoqué précédemment, certaines techniques d'encapsulation par voie chimique présentent l'inconvénient de ne pas permettre une encapsulation de composés liquides ou gazeux et surtout une quasi impossibilité d'obtenir, de façon reproductible et contrôlée, des structures dont les dimensions sont de l'ordre du nanomètre.

Pour remédier à cela, la présente invention concerne un procédé permettant l'obtention d'une structure S de capsule nanométrique 4, comportant au moins une capsule de taille nanométrique 4, notamment apte à permettre une encapsulation d'un composé, et de préférence une pluralité de capsules nanométriques 4, comme illustré notamment sur la figure 2C.

Ainsi, une structure S comportant une seule capsule nanométrique 4 correspond à une capsule nanométrique 4 individuelle.

Une capsule nanométrique 4 individuelle peut également être obtenue à partir d'une structure S comportant une pluralité de capsules nanométriques 4 séparées les unes des autres par un moyen adapté.

Plus particulièrement, le procédé selon l'invention, schématisé sur les figures 2A, 2B et 2C, comporte une étape de dépôt d'au moins une couche de résine 5 sur une couche de substrat 6 suivie d'une étape au cours de laquelle ladite couche de résine 5 est structurée par lithographie électronique à faible tension ou par lithographie optique ou par nanoimpression, pour obtenir la formation de cavités 7 dans l'épaisseur de cette couche 5. De façon particulièrement avantageuse, la cavité 7 présente une profondeur p inférieure à l'épaisseur E de la couche de résine 5, de sorte à ne pas percer totalement l'épaisseur de la couche de résine 5.

Par la suite, comme visible sur la figure 2A, un dépôt d'au moins une couche d'un matériau encapsulant 8 est réalisé sur toute la surface de la résine 5 par pulvérisation cathodique sous vide, en utilisant un équipement de pulvérisation cathodique sous vide.

Un tel dépôt est qualifié de dépôt « isotrope ». En d'autres termes, cela signifie que le dépôt est continu sur l'ensemble de la surface de la couche de résine 5, notamment au niveau des parois latérales et du fond des cavités 7, résultant de l'étape de structuration de la résine 5, ainsi que sur le restant de la résine 5 qui n'a pas été structurée.

La ou les cavités 7, recouvertes de matériel encapsulant 8, peuvent ensuite avantageusement être remplies par un composé 14 quelconque que l'on souhaite encapsuler, comme illustré sur la figure 2B. Ledit composé 14 peut être indifféremment à l'état liquide, gazeux, ou solide. Le composé encapsulé peut encore consister en du vide, dont la pression est inférieure à la pression atmosphérique. Une telle opération est effectuée par dépôt dudit composé 14 au sein de la cavité 7, par tout moyen connu de l'homme du métier et adapté à cet effet.

La ou les cavités 7, comportant à présent éventuellement, mais non obligatoirement, un composé 14 adéquat, sont alors obturées par l'intermédiaire d'un matériau d'obturation 10, ce dernier étant déposé sur l'ensemble de la surface, par-dessus la couche de matériau encapsulant 8 et par-dessus les cavités 7 contenant optionnellement le composé 14. Cette étape du procédé est illustrée sur la figure 2C ci-jointe.

Une fois cette opération réalisée, on obtient, au sein de la couche de résine 5, une structure S de capsule nanométrique 4, apte à permettre l'encapsulation d'un composé, et comportant préférentiellement une pluralité de capsules nanométriques 4. Une telle structure S de capsule nanométrique 4 peut également être désignée sous le terme de nanoréservoir ou nanostructure, ladite structure S de capsule nanométrique 4 étant obturée et pouvant renfermer, au sein des capsules 4, un composé 14 à encapsuler.

Par la suite, la couche de résine 5 est dissoute par trempage de cette dernière dans un solvant adéquat.

Les capsules nanométriques 4 de la structure S de capsule nanométrique peuvent alors être libérées de ladite résine 5. Lorsqu'une structure S comportant plusieurs capsules nanométriques 4 a été obtenue par le procédé selon l'invention, il est alors possible de séparer lesdites capsules 4 par agitation ; on peut ainsi obtenir des capsules nanométriques 4 individuelles en solution, celles-ci comportant avantageusement un composé 14 quelconque à l'intérieur.

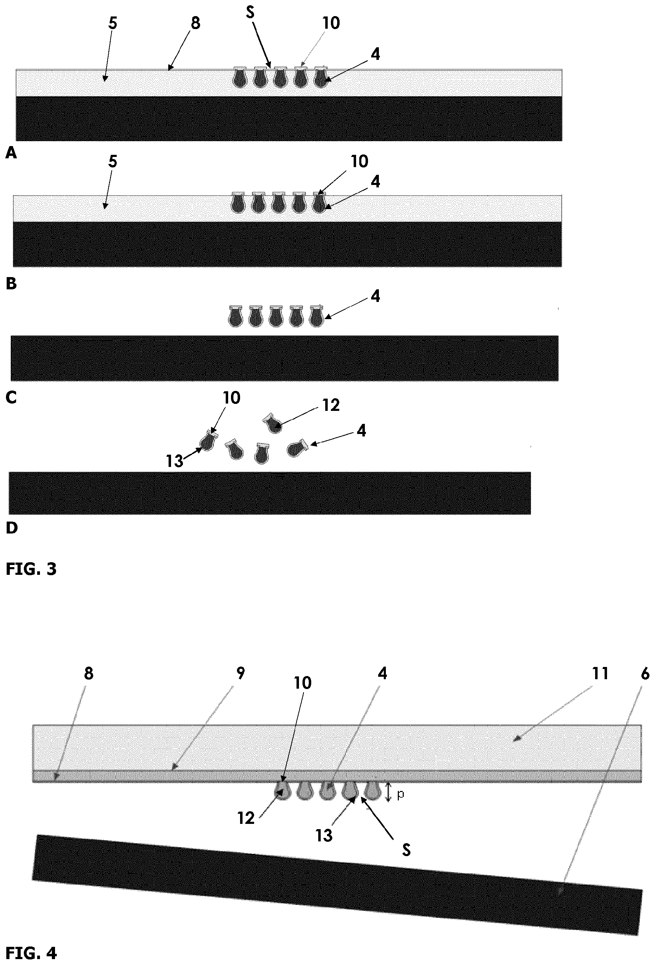

Un mode de réalisation particulier, permettant également l'obtention de capsules nanométriques 4 en solution, est illustré sur les figures 3A à 3D.

Dans ce mode de réalisation, après le dépôt d'une couche du matériau d'obturation 10, cette couche est structurée préférentiellement par la technique de lithographie électronique ; cependant, les techniques de lithographie optique ou de nanoimpression peuvent également être mises en oeuvre pour la structuration de la couche du matériau d'obturation 10. Quelle que soit la technique mise en oeuvre pour structurer la couche de matériau d'obturation 10, celle-ci est alignée avec la première lithographie qui permet la structuration de la couche de résine 5, de manière à éliminer ledit matériau 10 de toute la surface de ladite résine 5, excepté au niveau des capsules 4 qui restent obturées.

Le résultat est visible sur la figure 3A : on obtient une structure S de capsule nanométrique 4 comportant une pluralité de capsules nanométriques 4 aptes à permettre l'encapsulation d'un composé et fermées par le matériau d'obturation 10, ce dernier ayant été éliminé du reste de la surface.

Le matériau encapsulant 8, visible sur la figure 3A, est ensuite éliminé de la surface de la résine 5 préférentiellement par la technique de gravure chimique, ou par trempage, ou par gravure par faisceau d'ions (IBE) ou par gravure ionique réactive (RIE). Ces techniques permettent une dissolution du matériau encapsulant 8 comme illustré sur la figure 3B. Le matériau d'obturation 10 restant est, quant à lui, préservé.

La résine 5 est ensuite dissoute par action d'un solvant adéquat, comme le montre la figure 3C, ce qui permet une libération des capsules nanométriques 4 individuelles en solution. Cette étape est illustrée sur la figure 3D. Les capsules nanométriques 4 libérées comprennent au moins une capsule formée par un contenant 12 d'une profondeur p donnée, équivalente à celle de la cavité 7. Ledit contenant 12 comporte une enveloppe 13, formée par au moins une couche matériau encapsulant 8, et présente une ouverture refermée par un opercule, formé par le matériau d'obturation 10. Les capsules nanométriques 4 peuvent également comporter un composé 14 quelconque, encapsulé à l'intérieur desdites capsules 4.

Il est également possible, selon un mode de réalisation particulier du présent procédé représenté sur la figure 4, de transférer la structure S de capsule nanométrique 4 apte à encapsuler un composé sur un second substrat 11, préalablement à la dissolution de la couche de résine 5 dans un solvant.

Selon un mode de réalisation, pour transférer la structure S de capsule nanométrique 4, encapsulant éventuellement un composé 14, sur un second substrat 11, une résine de collage 9 peut être déposée par-dessus la couche de matériau d'obturation 10.

Selon un autre mode de réalisation, la résine de collage 9 peut être déposée directement par-dessus le matériau encapsulant 8. Dans ce cas, la résine de collage 9 remplit également la fonction de matériau d'obturation 10.

Dans certains cas, la couche de matériau d'obturation 10 peut également servir pour le collage lors du transfert la structure S de capsule nanométrique 4 sur un second substrat 11. On comprend donc que, dans le cas d'un transfert de ladite structure S de capsule nanométrique 4 sur un second substrat 11, la couche de matériau d'obturation 10 peut constituer la résine de collage 9.

Ainsi, le collage s'obtient par le dépôt du second substrat 11 sur la résine de collage 9 ou directement sur la couche de matériau d'obturation 10 et éventuellement par l'application d'un léger pressage.

Dans le cas d'un transfert de la structure S de capsule nanométrique 4, apte à permettre l'encapsulation d'un composé, sur un second substrat 11, on obtient les structures S telles que représentées sur les figures 5A et 5B jointes, c'est-à-dire associées à un second substrat 11 représenté sur la figure 5A. Au niveau de cette figure, le composé 14 éventuellement encapsulé dans la structure S de capsule nanométrique 4 est également illustré de façon schématique.

Pour en revenir à présent à la couche de substrat 6, sur laquelle est déposée la résine 5 au cours de la première étape du procédé selon l'invention, celle-ci peut être constituée par tous types de matériaux, qu'ils soient rigides ou non.

Ladite couche de substrat 6 peut par exemple être constituée par un matériau semi-conducteur, comme le silicium (Si), l'oxyde de zinc (ZnO) le nitrure d'aluminium (AlN).

Le substrat 6 peut également consister en un matériau isolant choisi notamment parmi l'oxyde de silicium (SiO), l'oxyde d'aluminium (Al₂O₃) ou encore le verre.

Le substrat 6 peut encore être constitué par un matériau plastique préférentiellement souple, comme par exemple le poly(méthyle méthacrylate), ou encore par un polymère.

L'emploi d'une couche de substrat 6, celle-ci présentant avantageusement une surface parfaitement plane, permet un dépôt précis et uniforme en épaisseur d'une couche de résine 5 présentant alors également une planéité et une régularité optimales. Cela garantit, dans la suite du procédé selon l'invention, une excellente reproductibilité lors de la structuration d'une résine 5, notamment lorsque celle-ci présente une surface importante, ainsi qu'un contrôle scrupuleux de la taille des capsules nanométriques 4 de la structure S obtenues lors de la mise en oeuvre du procédé selon l'invention.

En effet, l'utilisation de plaques de résine qui seraient imparfaitement planes, et qui présenteraient une rugosité de l'ordre du micron, ne permettrait pas de contrôler précisément la taille des capsules, ni d'obtenir des capsules de taille inférieure au micron.

Le dépôt de la résine 5 sur la couche de substrat 6 est avantageusement effectuée par la technique d'enduction centrifuge, également dénommée « spin coating » en anglais.

Dans cette technique, la couche de substrat 6 est posée et maintenue par le vide sur un plateau qui tourne à une vitesse importante et constante pour permettre un étalement du matériau déposé, en l'occurrence la résine 5, de façon parfaitement uniforme et contrôlée, sous l'effet de la force centrifuge.

En ce qui concerne à présent la résine 5, qui est déposée sur la couche de substrat 6, celle-ci est préférentiellement constituée de poly(méthyle méthacrylate) (PMMA) et/ou de poly(méthyle méthacrylate acide méthacrylique) (PMMA-MA). Ces composés sont des thermoplastiques transparents ; plus particulièrement, le PMMA consiste en un polymère transparent de méthacrylate de méthyle, également connu sous le nom de Plexiglas®, et qui présente une faible résistance aux solvants.

Cependant, ce mode de réalisation ne doit pas être compris comme étant limitatif de l'invention ; en effet, la couche de résine 5 peut également être constituée de toutes résines électrosensibles qui sont dissoutes par un solvant qui ne dissout pas la résine de collage 9, et notamment lorsqu'une étape de transfert des structures 4 sur un second substrat 11 est mise en oeuvre.

Un des points importants du procédé selon l'invention réside dans le fait que les cavités 7, obtenues lors de l'étape de structuration de ladite résine 5, doivent présenter une profondeur p inférieure à l'épaisseur E de la couche de résine 5, de manière à ne pas percer ladite couche de résine 5 et à faciliter, par la suite, la libération de la structure S de capsule nanométrique 4 par dissolution de la résine 5.

Ainsi, pour la structuration de la couche de résine 5, plusieurs techniques peuvent être employées ; préférentiellement, l'étape de structuration de la résine 5 est réalisée soit par lithographie électronique, soit par lithographie optique, ou encore par nanoimpression.

Selon un mode de réalisation particulièrement préférentiel du procédé selon l'invention, la structuration de la couche de résine 5 est effectuée en utilisant la technique de lithographie électronique. Plus préférentiellement encore, cette technique est mise en oeuvre dans des conditions particulières de manière à contrôler scrupuleusement la formation des cavités 7.

Ainsi, avantageusement, la lithographie électronique est mise en oeuvre à faible tension, par exemple entre 1 et 15 kV, par rapport aux tensions d'accélération qui sont usuellement utilisées en lithographie électronique (supérieures à 30kV) pour la structuration d'une résine et l'obtention de cavités ayant des dimensions de l'ordre du nanomètre. Une faible tension d'accélération est particulièrement avantageuse pour contrôler la pénétration du faisceau d'électrons dans la couche de résine 5 et ainsi la profondeur p de la/des cavité(s) 7 dans l'épaisseur E de ladite résine 5, de manière à ce que la/les cavité(s) 7 n'atteigne(nt) pas la couche de substrat 6.

Les inventeurs ont également mis en évidence qu'une faible tension d'accélération du faisceau d'électron, combinée à une épaisseur adéquate de la couche de résine 5, permettait de faciliter, de façon particulièrement intéressante, le contrôle de la pénétration des électrons dans ladite résine 5. De cette manière, il est encore plus aisé d'obtenir des cavités 7 qui n'atteignent pas la couche de substrat 6. En effet, dans le cas où la profondeur p de la cavité 7 correspondrait à l'épaisseur E de la résine 5, il s'avérerait extrêmement difficile, une fois le matériau encapsulant 8 et le matériau d'obturation 10 déposés, de séparer les capsules nanométriques 4 de la structure S, ces dernières étant alors reliées à la couche de substrat 6.

Préférentiellement, la couche de résine 5 présente une épaisseur E comprise entre 300nm et quelques micromètres, par exemple 2pm et la profondeur p des cavités 7 est comprise entre 20nm et 1µm, de préférence entre 50nm et 500nm et plus préférentiellement encore entre 50 et 300nm.

Cependant, un tel mode de réalisation n'est pas limitatif de l'invention, et la couche de résine 5 peut présenter une épaisseur E quelconque, tant que la cavité 7, obtenue lors de l'étape de structuration de ladite résine 5, présente une profondeur p inférieure à ladite épaisseur E.

Lors de la structuration de la couche de résine 5 par la technique de lithographie électronique, la dose d'électrons envoyée sur ladite résine 5 est également un paramètre important pour éviter de structurer cette résine 5 jusqu'à la couche de substrat 6, autrement dit pour éviter un percement de la couche de résine 5 jusqu'à la couche de substrat 6. Cependant, cette dose d'électrons que l'on peut envoyer au moment de la structuration dépend notamment de la constitution de la couche de résine 5, de la densité des cavités 7 au niveau de la résine 5 et des dimensions desdites cavités 7.

Ainsi, de façon intéressante, préalablement à l'étape de structuration par lithographie électronique, on réalise un test d'approche itérative pour déterminer la dose d'électrons qu'il est possible d'envoyer sur la couche de résine 5, de manière à éviter un percement de ladite couche de résine 5 jusqu'à la couche de substrat 6.

Pour cela, il est notamment possible d'utiliser un processus dit de « variation de dose » : à partir de deux valeurs extrêmes qui ont été estimées au préalable, on envoie une quantité d'électrons croissante sur la couche de résine 5. Après une étape de transfert sur un second substrat 11, on observe au microscope électronique à balayage si les capsules 4 obtenues sont détériorées ou non, en fonction de la dose d'électrons reçue. Si elles sont effectivement détériorées, cela signifie que la dose d'électrons envoyée au moment de l'étape de lithographie électronique est trop importante et que la totalité de l'épaisseur E de la couche de résine 5 a été percée.

Préférentiellement, la dose d'électrons qu'il est possible d'envoyer au moment de la structuration de la couche de résine 5 par lithographie électronique est inférieure ou égale à 250µC/cm², de préférence inférieure à 150µC/cm².

Une fois les cavités 7 creusées dans la couche de résine 5, le dépôt isotrope d'au moins une couche de matériau encapsulant 8 sur l'ensemble de la surface de ladite couche de résine 5 est effectué. De façon avantageuse, un tel dépôt isotrope est réalisé par pulvérisation.

Plus précisément, la technique de pulvérisation utilisée dans le procédé selon l'invention est la pulvérisation cathodique (ou « sputtering » en anglais) qui est effectuée sous vide.

La pulvérisation cathodique est une technique qui permet la synthèse de matériaux, à partir de la condensation d'une vapeur métallique issue d'une source solide, sur un substrat.

Les demanderesses ont mis en évidence qu'une telle technique permettait le dépôt isotrope d'au moins une couche mince et d'épaisseur contrôlée d'un matériau métallique ou semi-conducteur ou piézoélectrique en tant que matériau encapsulant 8.

Toute la difficulté à surmonter pour arriver à réaliser un dépôt isotrope de matériau encapsulant 8 résidait en effet dans le fait de recouvrir de façon uniforme et continue toute la surface de la cavité 7, et notamment les flancs inversés, ou parois latérales, de cette dernière. De plus, une exposition de la couche de résine 5 aux électrons et aux photons lors de l'étape de pulvérisation est susceptible de dégrader ladite résine 5. En conséquence, la durée pendant laquelle la résine 5 est exposée aux électrons doit être réduite, typiquement inférieure à 5min, et de préférence inférieure à 1min.

L'altération de la couche de résine 5, par insolation de cette dernière par des photons et/ou des électrons, peut être problématique car elle affecte l'étape ultérieure de dissolution de ladite résine 5, permettant la libération de la structure S de capsule nanométrique 4 ou des capsules nanométriques 4 elles-mêmes. En effet, lorsque la couche de résine 5 est altérée, sa dissolution par trempage dans un solvant n'est plus possible et il est alors nécessaire de procéder à un traitement ultrasonique. Un tel traitement présente cependant l'inconvénient d'endommager la structure S de capsule nanométrique 4 et est donc préférentiellement à éviter.

En conséquence, pour éviter une altération de la couche de résine 5 lors de l'étape de pulvérisation cathodique sous vide, celle-ci est préférentiellement réalisée sous une puissance modérée, comprise entre 80 et 120 Watts, et plus préférentiellement encore de l'ordre de 100W. En particulier, une puissance modérée est appliquée, lorsque l'on pulvérise une couche de matériau métallique.

Il est également avantageux de pulvériser le matériau encapsulant 8 suffisamment loin de l'échantillon 21, représenté sur la figure 2A, comportant la couche de substrat 6 et la résine 5. De plus, ledit échantillon 21 doit également être protégé de toute contamination particulaire, notamment par des électrons et/ou par des photons, avant ou après le dépôt de matériau encapsulant 8. Avantageusement, pour pallier au risque de contamination, un jeu de cache de taille suffisante peut être mis en place ce qui permet d'éviter toute insolation de la résine 5 notamment pendant la période de réglage du plasma de la pulvérisation.

Durant la mise en oeuvre du procédé selon l'invention, la couche de résine 5 est également susceptible de subir une dégradation du fait d'une élévation locale de la température, ce qui rendrait également difficile la dissolution ultérieure de ladite résine 5 par un solvant ; ainsi, préférentiellement, la température à laquelle est exposée la résine 5 est contrôlée pendant toute la durée du procédé. Plus préférentiellement encore, la température est maintenue entre 15°C et 30°C, de préférence entre 18 et 25°C, et typiquement aux alentours de 20°C.

L'application de ces conditions particulières facilite grandement le dépôt de matériau encapsulant 8 ; il est ainsi possible d'obtenir au moins une mince enveloppe de cet encapsulant 8 sur toute la surface de la résine 5, et notamment sur les flancs inversés des cavités 7. En appliquant le procédé selon l'invention, l'épaisseur e d'une couche de matériau encapsulant 8 peut être scrupuleusement contrôlée, et l'on peut alors obtenir une couche de taille nanométrique, dont l'épaisseur est typiquement comprise entre 10 et 200nm.

Il est également possible de pulvériser plusieurs couches de matériau encapsulant 8, chacune des couches présentant alors une épaisseur contrôlée comprise entre 10 et 200nm.

A propos du matériau encapsulant 8, ce dernier peut consister en tout type de matériau pulvérisable.

Préférentiellement, ledit matériau encapsulant 8 est choisi parmi les matériaux métalliques, comme l'aluminium (Al), le platine (Pt), le tantale (Ta), l'or (Au). Le matériau encapsulant 8 peut également être choisi parmi les matériaux semi-conducteurs, comme le silicium (Si), le germanium (Ge), le phosphure d'indium (InP) ou encore piézoélectriques, tels que notamment le nitrure d'aluminium (AlN), l'oxyde de zinc (ZnO), le nitrure de galium (GaN), le tantalate de lithium (LiTaO₃), le niobate de potassium (KNbO₃) ou encore le niobate de lithium (LiNbO₃) ; les matériaux piézoélectriques présentent notamment la propriété de se déformer lorsqu'on les soumet à un champ électrique. En conséquence, une structure S de capsule nanométrique 4 obtenue à partir d'un matériau encapsulant 8 de type piézoélectrique pourra être rompue par application d'un champ électrique et ainsi libérer le composé 14 éventuellement encapsulé à l'intérieur de la cavité 7.

Enfin, le matériau encapsulant 8 peut consister en un matériau isolant, tel que notamment l'oxyde de silicium (SiO₂), le nitrure de silicium (Si₃N₄) ou l'alumine (Al₂O₃) ou encore le verre.

L'or, le platine et le tantale sont particulièrement intéressants car ils sont considérés comme étant des matériaux biocompatibles. On qualifie de « biocompatible » un matériau qui n'interfère pas et ne dégrade pas le milieu biologique dans lequel il est utilisé.

Avantageusement, dans le procédé selon l'invention, l'on peut pulvériser successivement plusieurs couches comportant chacune un matériau encapsulant.

Ainsi, il est avantageux de pulvériser, dans un premier temps, dans la cavité 7, une couche 8 d'un matériau métallique, notamment Al, Pt, Ta, Si ou Au, puis, dans un second temps, une couche de matériau piézoélectrique (ZnO, AlN, GaN, LiTaO₃, KNbO₃ LiNbO₃) et enfin pour terminer, une troisième couche d'un matériau métallique tel que défini ci-dessus.

Une telle séquence de pulvérisation permet de fabriquer par exemple une structure S de capsule nanométrique 4 ayant une enveloppe 13 comportant une couche d'un matériau piézoélectrique, entourée par deux couches d'un matériau métallique, ladite structure S de capsule nanométrique 4 étant alors apte à générer de l'énergie électrique par interaction entre le métal et le matériau piézoélectrique, cette énergie étant susceptible d'être récupérée.

Une fois l'étape de dépôt par pulvérisation d'au moins une couche d'un matériau encapsulant 8 achevée, chacune des cavités 7 peut être avantageusement remplie d'un composé 14, représenté notamment sur la figure 2B, qui peut être solide, liquide ou gazeux. La cavité 7 peut également être remplie par du vide, dont la pression est inférieure à la pression atmosphérique.

La ou les cavités 7 sont ensuite obturées par l'intermédiaire d'un matériau d'obturation 10, ce dernier permettant de refermer la ou lesdites cavités 7 pour obtenir une structure S de capsule nanométrique 4 incorporant ledit composé 14.

Le matériau d'obturation 10 peut être de composition quelconque, notamment souple ou rigide. Il peut ainsi être d'une composition identique, ou différente, de celle du matériau encapsulant 8 ou de la résine de collage 9.

Le matériau d'obturation 10 peut avantageusement consister en un polymère ou en un métal, ou encore en une résine **époxyde**. Il est cependant préférentiel que ledit matériau d'obturation 10 soit résistant au solvant qui est utilisé lors de l'étape de dissolution de la couche de résine 5. En effet, on comprend aisément que, si tel n'est pas le cas, il est impossible d'obtenir une structure S dont les capsules nanométriques 4 sont fermées.

De plus, il est également avantageux que le matériau d'obturation 10 utilisé dans le procédé selon l'invention permette un collage de la structure S de capsule nanométrique 4 sur un second substrat 11, dans le cas notamment où l'on procède à une étape de transfert de ladite structure S de capsule nanométrique 4 sur un second substrat 11. Lorsque le matériau d'obturation 10 permet le collage lors de l'étape de transfert, le dépôt d'une résine de collage 9 supplémentaire n'est alors pas nécessaire. Le matériau d'obturation 10 constitue dans ce cas la résine de collage permettant le transfert de la structure S de capsule nanométrique 4 sur un second substrat 11.

Ledit matériau d'obturation 10 peut préférentiellement consister en une résine biocompatible, notamment la résine photosensible de type SU8 constituée d'un polymère visqueux de type époxyde.

Une telle résine SU8 utilisée en tant que matériau d'obturation 10 est particulièrement avantageuse ; d'une part, comme mentionnée précédemment, la résine SU8 est biocompatible ce qui permet de l'utiliser pour la fabrication de structures S de capsule nanométrique 4 destinées à des applications biomédicales. D'autre part, une telle résine SU8 peut servir au moment du collage lors du transfert de la structure S de capsule nanométrique 4 sur un second substrat 11. L'utilisation de la SU8 permet ainsi d'éviter le dépôt d'une résine spécifiquement destinée à permettre le collage au moment du transfert ; la résine SU8 a notamment été utilisée lors de l'obtention de la structure S de capsule nanométrique 4 visible sur la figure 5B et de la capsule nanométrique 4 individuelle de la figure 5C.

Le dépôt dudit matériau d'obturation 10, qui peut notamment consister en une résine biocompatible de type SU8, peut être effectué par tout moyen connu de l'homme du métier et apte à cet effet.

Préférentiellement, ce dépôt peut être effectué par la technique d'enduction centrifuge, également appelée « spin coating » mentionnée précédemment.

Selon un autre mode de réalisation, le dépôt du matériau d'obturation 10 est effectué par la technique de projection, également dénommée « spray coating » : cette technique met en oeuvre un système de projection utilisant un gaz vecteur dans le but d'accélérer et de transférer des fines particules du matériau d'obturation 10 à l'endroit désiré.

Une autre technique pouvant être utilisée est le trempage, également connu sous le nom de « dip coating ». Cette technique consiste à mettre en forme une couche par immersion de l'ensemble comportant le substrat 6 et la résine 5, structurée et au niveau de laquelle a été déposée une couche de matériel encapsulant 8, dans une cuve contenant le matériau d'obturation 10.

Pour en revenir à présent au solvant utilisé pour permettre la dissolution de la couche de résine 5, celui-ci consiste préférentiellement en de l'acétone, notamment lorsque la résine 5 est constituée par du poly(méthyle méthacrylate). Cependant, ce mode de réalisation particulier n'est pas limitatif de l'invention, et il est aisément imaginable d'utiliser d'autres solvants adaptés à la dissolution de la résine 5, en fonction de la composition de cette dernière.

Le procédé selon la présente invention permet ainsi d'obtenir une structure S de capsule nanométrique 4 comportant au moins une capsule 4 de taille nanométrique apte à permettre l'encapsulation d'un composé et comportant un matériau d'encapsulation 8, remplie optionnellement d'un composé 14 quelconque, et refermée par un matériau d'obturation 10.

Plus particulièrement, la/les capsule(s) nanométrique(s) 4 comprend/comprennent au moins une capsule 4 formée par un contenant 12 d'une profondeur p donnée, équivalente à celle de la cavité 7. Ledit contenant 12 comporte une enveloppe 13, formée par au moins une couche de matériau encapsulant 8, et présente une ouverture refermée par un opercule, formé par le matériau d'obturation 10.

Préférentiellement, la couche de matériau encapsulant 8 de ladite enveloppe 13 présente une épaisseur e qui respecte un certain rapport p/e, avec p correspondant à la profondeur du contenant 12 ; ainsi, avantageusement, p/e est supérieur ou égal à 2, de préférence supérieur à 6 et, plus préférentiellement encore, p/e est supérieur à 10.

Sur la figure 5B, la structure S de capsule nanométrique 4 qui a été obtenue et qui est observée au microscope électronique à balayage comporte des capsules nanométriques 4 qui présentent une profondeur p sensiblement égale à 330nm et une épaisseur e de l'ordre de 30nm. Ainsi, le rapport p/e est environ égal 11.

La figure 5C montre une capsule nanométrique 4 unique, également photographiée au microscope électronique à balayage. La profondeur p est de l'ordre de 800nm et le diamètre est de l'ordre de 300nm. L'opercule, formé par le matériau d'obturation 10, en l'occurrence la résine SU8, est bien visible sur cette figure.

Les capsules nanométriques 4, encapsulant un composé quelconque, sont également représentées schématiquement sur la figure 3D, lorsqu'elles sont libres en solution, et sur la figure 4, lorsqu'elles sont associées à un second substrat 11.

De façon particulièrement avantageuse, la profondeur p du contenant 12 est comprise entre 20nm et 1µm, de préférence entre 50 et 500nm et plus avantageusement encore entre 50 et 300nm.

En effet, en appliquant le procédé selon l'invention, et notamment en contrôlant la pénétration du faisceau au moment de l'étape de structuration de la couche de résine 5, il est aisé d'obtenir une capsule nanométrique 4 dont le contenant 12 présente la profondeur p voulue, cette dernière pouvant varier notamment en fonction du composé 14 qui peut être encapsulé dans ladite capsule 4.

En ce qui concerne à présent l'enveloppe 13, celle-ci comporte au moins une couche d'un matériau encapsulant 8. L'épaisseur e d'une couche de matériau encapsulant 8 respecte le rapport suivant : p/e supérieur à 2, de préférence supérieur à 6 et de préférence supérieur à 10.

Lorsque l'enveloppe 13 comporte une pluralité de couches de matériau encapsulant, chacune des couches présente une épaisseur e respectant le rapport p/e tel que défini dans le paragraphe précédent.

De préférence, l'épaisseur d'une couche de matériau encapsulant 8 est avantageusement comprise entre 10 et 200nm. Ainsi, les capsules nanométriques 4 de la structure S aptes à permettre l'encapsulation d'un composé présentent une mince enveloppe 13, comportant une ou plusieurs couches de matériau encapsulant 8, entourant éventuellement le composé encapsulé 14. Cela permet de protéger de façon efficace ledit composé 14, tout en facilitant la libération de celui-ci au moment opportun.

Les capsules nanométriques 4 de la structure S, obtenue par la mise en oeuvre du procédé selon l'invention, présentent préférentiellement une forme sensiblement cylindrique avec une base sensiblement ronde. Dans ce cas de figure, leur diamètre est variable et peut être compris entre 50nm et quelques microns. Cependant, comme illustré sur les figures jointes, lesdites capsules nanométriques 4 peuvent également être légèrement évasées en allant vers la partie de la résine 5 au contact de la couche du substrat 6, présentant alors une forme proche de celle d'une goutte. Les capsules nanométriques 4 de la structure S peuvent également présenter une base quelconque, et notamment de forme triangulaire ou rectangulaire.

La structure S de capsule nanométrique 4 selon la divulgation est notamment utilisée pour encapsuler un composé 14, ce dernier pouvant être quelconque. En fonction de la nature du composé 14, ladite structure S de capsule nanométrique 4 peut avoir des applications diverses, dans des domaines différents.

Comme évoqué plus haut dans la description, les capsules nanométriques 4 de la structure S, pouvant encapsuler un composé 14, sont susceptibles d'être libérées en solution, comme l'illustre la figure 3D. Dans un autre mode de réalisation, notamment représenté sur les figures 4, 5A et 5B, la structure S de capsule nanométrique 4 peut également être associée à un second substrat 11 suite à une étape de transfert par collage.

La structure S de capsule nanométrique 4 selon la divulgation, comportant au moins une capsule nanométrique 4, est particulièrement avantageuse, notamment parce qu'elle permet une libération sur commande du composé 14 encapsulé dans la/les capsule(s) nanométriques 4. Cette libération peut notamment être obtenue par l'application, au niveau de la structure S de capsule nanométrique 4, d'un moyen permettant la destruction de l'enveloppe 13, à un moment précis et choisi. Un tel moyen peut notamment consister en un courant électrique ou en un champ électromagnétique ou en un champ ultrasonique ou en un champ électrique. En fonction du ou des matériau(x) encapsulant 8 constituant l'enveloppe 13 de la structure S de capsule nanométrique 4, une telle application va entrainer une déstructuration et une rupture de ladite enveloppe 13, ce qui va aboutir à une libération du composé 14 enfermé dans la/les capsule (s) nanométrique(s) 4 de la structure S.

En particulier, lorsque le matériau encapsulant 8 est constitué par un matériau piézoélectrique, la structure S de capsule nanométrique 4 est soumise à un champ électrique pour entrainer une libération du composé 14 encapsulé.

De façon avantageuse, il est possible d'utiliser ces moyens, permettant une libération du composé 14, que la structure S de capsule nanométrique 4 soit associée à un substrat ou que les capsules nanométriques 4 soient libres en solution.

Dans ce dernier cas, la structure S de capsule nanométrique 4 peut par exemple être transférée à la surface d'un substrat 11 qualifié d'« intelligent » dont les propriétés intrinsèques permettent une libération conditionnelle dudit composé encapsulé 14 lorsque des contraintes d'ordre chimique et/ou thermique et/ou mécanique et/ou biologique sont appliquées audit substrat intelligent.

Une première application d'une structure S de capsule nanométrique 4 selon la divulgation est relative à l'incorporation d'un composé 14 de type lubrifiant à l'intérieur de ladite structure S, au sein des capsules nanométriques 4. Ces dernières peuvent alors être disposées sur une surface nécessitant une lubrification permanente. Une telle surface peut notamment consister en un système de sécurité dans un satellite, ce dernier étant donc destiné à être utilisé en milieu hostile, par exemple dans l'espace, dans lequel le composé lubrifiant s'évapore rapidement de la surface. Dans cette application particulière, illustrée de façon schématique sur la figure 6 annexée, la structure S de capsule nanométrique 4 maintient le composé lubrifiant à la surface du système, évitant ainsi une évaporation dudit composé 14 dans l'espace. L'utilisation d'une structure S de capsule nanométrique 4 selon l'invention permet en conséquence de garantir une lubrification optimale, et sur une longue période, de la surface sur laquelle elles sont disposées. La libération du composé lubrifiant hors des capsules nanométriques 4 de la structure S peut alors être effectuée sur commande, par exemple par un appareillage 15, pouvant consister en un racleur, qui entraine une destruction desdites capsules nanométriques 4 et une libération du composé sur la surface à lubrifier.

Selon une seconde application particulière de l'invention, la structure S de capsule nanométrique 4 est destinée à une application biomédicale. Dans ce cas, le composé encapsulé 14 dans les capsules nanométriques 4 consiste en au moins un principe actif 16 d'un médicament. On entend par principe actif d'un médicament l'ensemble des composants de ce médicament présentant un effet thérapeutique.

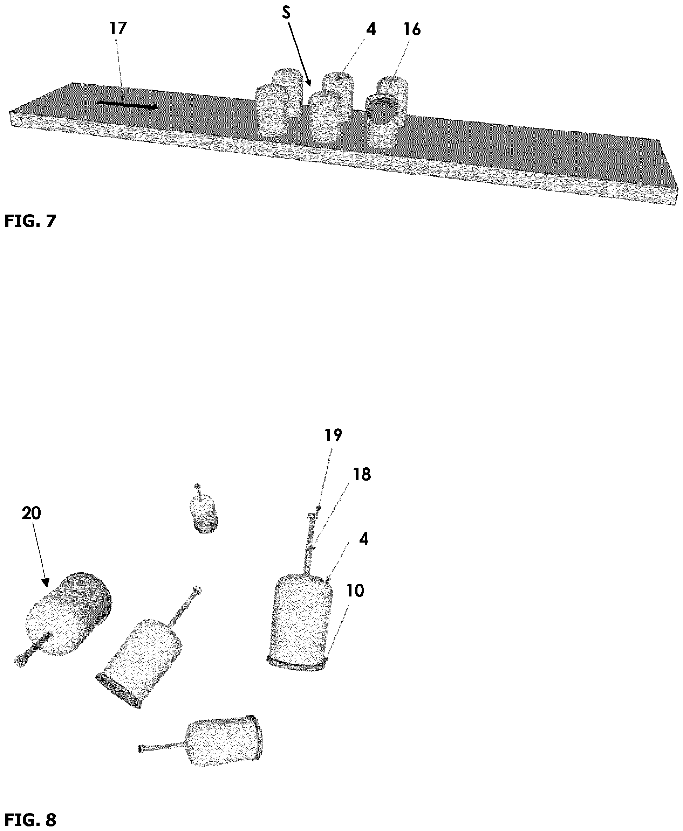

En particulier, sur la figure 7, un principe actif 16 est encapsulé dans les capsules 4 d'une structure S disposée à la surface d'un substrat conducteur dans lequel circule un courant 17 entraînant, par dilatation et hyperthermie, une libération dudit principe actif 16 au moment opportun. La circulation du courant 17 dans le substrat conducteur permet donc une libération sur commande du principe actif 16 encapsulé.

Selon un autre mode de réalisation particulier, représenté sur la figure 8, des capsules nanométriques 4, obtenues par exemple par libération à partir d'une structure S comportant une pluralité de capsules 4, et incorporant un principe actif non visible sur cette figure, sont couplée à une séquence thiol-antigène ou à une séquence thiol-anticorps pour une application biomédicale. Dans ce cas précis, le matériau d'encapsulation 8 dont sont constituées les capsules nanométriques 4 est préférentiellement de l'or, du platine ou du tantale, du fait de la biocompatibilité de ces métaux. En ce qui concerne le matériau d'obturation 10, celui-ci consiste préférentiellement en une résine biocompatible de type SU8.

Le groupement thiol 18 permet de faire la liaison entre la capsule nanométrique 4 et l'anticorps ou l'antigène 19. Ce dernier peut cibler de façon spécifique une cellule indésirable présente dans le corps humain, notamment une cellule cancéreuse. Le complexe 20 comportant la capsule nanométrique 4 et la séquence thiol-antigène ou anticorps, se fixe donc sur la cellule cible à éliminer. Ladite cellule cible, sur laquelle est fixée spécifiquement le complexe 20, est alors détruite, par application d'un champ électromagnétique par exemple. En effet, un tel champ permet une désintégration de la capsule nanométrique 4 qui entraine également la destruction, par hyperthermie, de la cellule cible sur laquelle ladite capsule 4 est fixée par l'intermédiaire de la séquence thiol-antigène ou anticorps. La capsule nanométrique 4 ainsi rompue libère alors le principe actif qui va se répandre au voisinage de la cellule cible qui a été détruite.

De façon particulièrement avantageuse, les capsules nanométriques 4 selon la divulgation permettent, en plus de faciliter la destruction de cellules indésirables, de diffuser un principe actif adapté au niveau de la zone qui doit être traitée.

De nombreuses autres applications, mettant en oeuvre la structure S de capsule nanométrique 4 ou les capsules nanométriques 4 selon la divulgation, sont envisageables.

Par exemple, une structure S comprenant une grande densité de capsules nanométriques 4 réalisée à partir d'un matériau encapsulant 8 métallique permet de réaliser un miroir réfléchissant à tout angle d'incidence.

Selon un autre mode de réalisation, si le matériau encapsulant 8 utilisé pour la fabrication de la structure S de capsule nanométrique 4 consiste en du verre, et que le composé 14 encapsulé consiste en un gaz, notamment l'ozone (O₃) présentant la propriété d'absorber les ultraviolets, il est possible de fabriquer des miroirs réfléchissant certaines longueurs d'ondes et absorbant d'autres longueurs d'ondes.

En particulier, de telles applications sont possibles lorsque les capsules nanométriques 4 de la structure S présentent une géométrie courbe.

Selon une autre application de la structure S de capsule nanométrique 4, lorsque les capsules nanométriques 4 présentent une base rectangulaire, il est possible de fabriquer des structures lasantes. En particulier, pour cette application, le composé encapsulé est préférentiellement un gaz, notamment l'hélium-néon (HeNe), et la structure S de capsule nanométrique 4 est préférentiellement constituée par du verre et du métal.

Dans une autre application intéressante des structures S de capsule nanométrique 4 selon la divulgation, celles-ci incorporent des matériaux explosifs ou un comburant et sont dissoutes dans une solution de carburant. Cela permet avantageusement une augmentation de la poussée des avions ou des fusées.

Les structures S de capsule nanométrique 4 peuvent également renfermer un produit désinfectant ou un produit purificateur.

Une structure S de capsule nanométrique 4 selon la présente divulgation peut également servir de support pour réaliser des nanofils constitués par un matériau différent de celui desdites capsules 4. En particulier, les nanofils peuvent notamment être constitués d'or et les capsules nanométriques 4 d'aluminium. Les nanofils sont alors obtenus par évaporation à 45°C d'or et les capsules nanométriques 4 sont ensuite dissoutes. Il est alors possible d'obtenir des nanofils d'or qui sont libérables en solution.

Il est encore possible d'utiliser une structure S de capsule nanométrique 4 selon la divulgation dans des applications particulières permettant une génération et une récupération d'énergie.

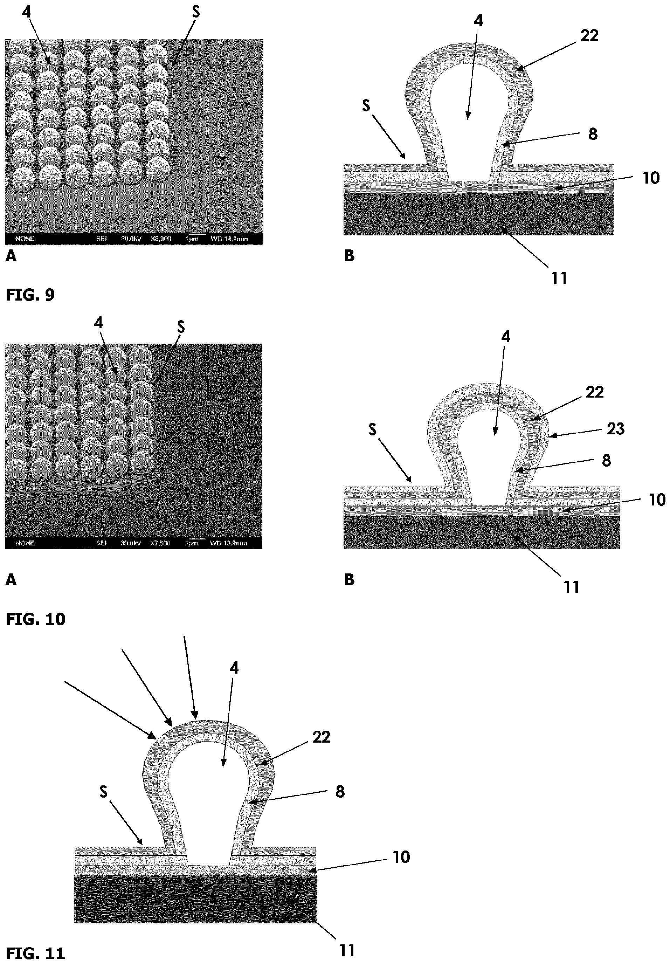

Ces applications sont illustrées notamment sur les figures 9 et 10.

Sur la figure 9B est illustrée schématiquement une capsule nanométrique 4 d'une structure S de capsule nanométrique 4 dont l'enveloppe comporte une première couche métallique 8, par exemple de l'aluminium et une seconde couche 22 d'un matériau piézoélectrique, par exemple l'oxyde de zinc.

Cependant, il est possible d'utiliser d'autres matériaux métalliques ou piézoélectrique qui ont déjà été détaillés précédemment.

La figure 9A correspond à une photographie prise au microscope électronique à balayage d'une telle structure S de capsule nanométrique 4 comportant une pluralité de capsules nanométriques 4.

Cette structure S de capsule nanométrique 4 représentée sur la figure 9A peut être obtenue selon deux approches. La première consiste à pulvériser les deux couches 8 et 22 par pulvérisation cathodique sous vide suite à la structuration de la résine 5 dans les cavités 7 obtenues, puis à transférer la structure S de capsule nanométrique 4 sur un second substrat 11 en utilisant soit un matériau d'obturation 10 pour permettre le collage de la structure S sur ledit second substrat 11, soit en utilisant une résine de collage, ce dernier mode de réalisation n'étant pas représenté.

La seconde approche consiste à pulvériser, toujours en utilisant la technique de pulvérisation cathodique sous vide, une première couche, en l'occurrence la couche métallique 8, puis de réaliser le transfert sur un second substrat 11, et enfin de pulvériser à nouveau, après l'étape de transfert, une couche 22 de matériau piézoélectrique par-dessus la première couche métallique 8. La seconde pulvérisation est également une pulvérisation cathodique sous vide.

Les figures 10A et 10B illustrent une structure S de capsule nanométrique 4 pouvant également être obtenue par les deux approches mentionnées ci-dessus pour l'obtention de la structure S de capsule nanométrique 4 de la figure 9, la structure S de capsule nanométrique 4 de la figure 10B comportant toutefois une couche supplémentaire 23 pouvant consister par exemple en une couche d'un matériau métallique.

Les structures S de capsule nanométrique 4 ainsi obtenues, comportant une couche de matériau métallique 8 et une couche de matériau piézoélectrique 22, ou encore deux couches métalliques 8 et 23 encadrant une couche d'un matériau piézoélectrique 22, permettent, lorsqu'elles sont sollicitées mécaniquement, une génération d'électricité.

En effet, les matériaux piézoélectriques ont la capacité de transformer une déformation mécanique en un champ électrique, et *vice versa.* Il est alors possible, avec les structures S de capsule nanométrique 4 représentée sur les figures 9 et 10 de capter une déformation mécanique et de générer de l'électricité.

De telles structures S de capsule nanométrique 4 peuvent ainsi trouver une application dans la fabrication de vêtements dits « intelligents ».

En ce qui concerne plus particulièrement la structure S de capsule nanométrique 4 représentée sur la figure 10B, et présentant une enveloppe comportant une couche 22 d'un matériau piézoélectrique encadrée par deux couches 8 et 23 d'un matériau métallique, celle-ci peut également permettre la réalisation de nano-résonateurs à ondes acoustiques, ces derniers entrant en résonance à leur fréquence propre lorsqu'ils sont soumis à un signal électrique contenant cette fréquence.

En effet, la fréquence de résonance est inversement proportionnelle à l'épaisseur de la couche 22 de matériau piézoélectrique. De ce fait, une couche 22 présentant une épaisseur comprise préférentiellement entre 10 et 200nm permet d'obtenir des fréquences de résonance allant du gigahertz au térahertz.

De tels nano-résonateurs, qui sont par nature miniatures, trouveront une application intéressante dans des systèmes de télécommunication, notamment les radars automobiles ou dans le domaine de l'aviation, ou encore dans le domaine de la téléphonie mobile.

La figure 11 illustre quant à elle une application d'une structure S de capsule nanométrique 4 obtenue par la mise en oeuvre du procédé selon l'invention dans le domaine optique.

Plus particulièrement, une structure S de capsule nanométrique 4 pouvant être utilisée dans le domaine de l'optique présente une première couche 8 d'un matériau métallique et une seconde couche d'un matériau semi-conducteur 22, tel que notamment l'oxyde de zinc.

Une structure S de capsule nanométrique 4 représentée schématiquement sur la figure 11 peut également être obtenue par la mise en oeuvre des deux approches, à savoir :
- pulvérisation des deux couches 8 et 22 après structuration de la résine 5 puis transfert sur un second substrat 11 par l'intermédiaire d'une couche 10 d'un matériau d'obturation ou d'une résine de collage non représentée sur la figure 11;
- ou pulvérisation d'une première couche 8 métallique, transfert de la structure S de capsule nanométrique 4 sur un second substrat 11 puis nouvelle étape de pulvérisation d'une seconde couche 22 d'un matériau semi-conducteur sur la première couche métallique 8.

La forme de la structure S de capsule nanométrique 4, et notamment la géométrie courbe des capsules nanométriques 4, se rapprochant de celle d'une goutte, permet d'obtenir une surface dont l'absorption ou l'émission de photon est nettement améliorée. En effet, quel que soit l'angle d'incidence des rayons lumineux sur les capsules nanométriques 4, lesdits rayons, représentés par les flèches sur la figure 11, arrivent sur la surface desdites capsules nanométriques 4 de la structure S de capsule nanométrique 4 en incidence normale. Cela permet avantageusement d'éviter les réflexions sur la surface lorsque l'angle d'incidence n'est pas normal au plan.

Lorsqu'une couche de matériau semi-conducteur 22 recouvre la structure S de capsule nanométrique 4, il est possible d'obtenir une surface absorbante de type photodiode quel que soit l'angle d'incidence des rayons, ou alors une surface émettrice selon une émission omnidirectionnelle.

En effet, les matériaux semi-conducteurs sont capables d'interagir avec la lumière, notamment avec les photons de la lumière et avec les électrons. Il est alors possible, avec une structure S de capsule nanométrique 4 présentant une première couche de matériau métallique 8 et une seconde couche 22 d'un matériau semi-conducteur, de fabriquer une photodiode apte à capter les photons et à générer de l'électricité.

L'utilisation de telles structures S de capsule nanométrique 4 permet en conséquence une amélioration considérable de l'efficacité des panneaux solaires.

Il est également envisageable de fabriquer une source optique omnidirectionnelle, de type diode électroluminescente ou laser, apte à capter les électrons et à générer des photons, donc à émettre de la lumière.

Eventuellement, pour de telles applications, la structure S de capsule nanométrique comporte, en plus des deux couches 8 et 22 susmentionnées, une troisième couche constituée par un matériau métallique, non représenté sur la figure 11, encadrant la couche en matériau semi-conducteur 22 avec la première couche métallique 8.

## Revendications

1. Procédé pour l'obtention d'au moins une structure (S) de capsule nanométrique (4) comportant au moins une capsule (4) de taille nanométrique apte à permettre l'encapsulation d'un composé, ledit procédé étant **caractérisé par le fait qu'**il comporte les étapes suivantes :
- on dépose au moins une couche de résine (5) sur une couche de substrat (6);
- on structure ladite couche de résine (5), par lithographie électronique à faible tension, ou par lithographie optique ou par nanoimpression de manière à obtenir au moins une cavité (7) dans l'épaisseur de ladite couche de résine (5), ladite cavité (7) présentant une profondeur p inférieure à l'épaisseur E de ladite couche de résine (5) ;
- on effectue un dépôt isotrope d'au moins une couche d'un matériau encapsulant (8) par pulvérisation cathodique sous vide dudit matériau (8);
- on obture la/les cavité(s) (7) avec un matériau d'obturation (10) ;
- on dissout la couche de résine (5) par trempage dans un solvant adapté.

2. Procédé selon la revendication 1 **caractérisé en ce que**, suite à l'étape de dépôt isotrope d'au moins une couche d'un matériau encapsulant (8) par pulvérisation cathodique sous vide, l'on remplit la/les cavité(s) (7) d'un composé (14) quelconque, ledit composé (14) pouvant être liquide, solide ou gazeux, y compris du vide.

3. Procédé selon l'une quelconque des revendications précédentes **caractérisé par le fait que** l'on structure la couche de résine (5) par lithographie électronique à faible tension, cette dernière étant comprise entre 1 et 15kV.

4. Procédé selon la revendication 3 **caractérisé par le fait que**, préalablement à la structuration de la couche de résine (5) par lithographie électronique, on réalise un test d'approche itérative pour déterminer la dose d'électrons à envoyer sur la couche de résine (5), de manière à éviter un percement de ladite couche de résine (5) jusqu'à la couche de substrat (6).

5. Procédé selon l'une quelconque des revendications précédentes **caractérisé par le fait que** l'on dépose, sur la couche de substrat (6), par enduction centrifuge, une couche de résine (5) présentant une épaisseur E comprise entre 300 nm et 2µm et que l'on structure ladite couche de résine (5) de manière à obtenir une cavité (7) présentant une profondeur p comprise entre 20nm et 1µm, de préférence entre 50 et 500nm, de préférence entre 50 et 300nm, ladite profondeur p étant inférieure à ladite épaisseur E.

6. Procédé selon l'une quelconque des revendications précédentes **caractérisé par le fait que** l'on effectue le dépôt isotrope par pulvérisation cathodique sous vide de matériau encapsulant (8) à une puissance comprise entre 80 et 120 Watts, préférentiellement égale à 100W.

7. Procédé selon l'une quelconque des revendications précédentes **caractérisé par le fait que** l'on effectue toutes les étapes dudit procédé à une température contrôlée comprise entre 15°C et 30°C, de préférence entre 18 et 25°C, de préférence égale à 20°C.

8. Procédé selon l'une quelconque des revendications précédentes **caractérisé par le fait que** l'on pulvérise au moins une couche de matériau encapsulant (8) dont l'épaisseur e est comprise entre 10 et 200nm.

9. Procédé selon l'une quelconque des revendications précédentes **caractérisé par le fait que** l'on dépose une couche de résine (5) constituée de poly(méthyle méthacrylate) et/ou de poly(méthyle méthacrylate acide méthacrylique).

10. Procédé selon l'une quelconque des revendications précédentes **caractérisé par le fait que** le substrat (6) est constitué par un matériau souple ou rigide choisi parmi le silicium, l'oxyde de zinc, le nitrure d'aluminium, l'oxyde de silicium, l'oxyde d'aluminium, les polymères, les matériaux plastiques.

11. Procédé selon l'une quelconque des revendications précédentes **caractérisé par le fait que** l'on dépose un matériau encapsulant (8) pulvérisable choisi parmi les matériaux métalliques, les matériaux semi-conducteurs les matériaux piézoélectriques et les matériaux isolants.

12. Procédé selon l'une quelconque des revendications précédentes **caractérisé par le fait que** l'on obture la/les cavité(s) (7) avec un matériau d'obturation (10) choisi parmi les polymères, les métaux, les résines époxydes et les résines biocompatibles, ledit matériau d'obturation (10) étant apte à résister au solvant utilisé dans l'étape de dissolution de la résine (5).

13. Procédé selon l'une quelconque des revendications précédentes **caractérisé par le fait qu'**il comporte encore une étape, préalable à l'étape de dissolution de la couche de résine (5), au cours de laquelle on transfère la structure (S) de capsule nanométrique (4) sur un second substrat (11) en appliquant une résine de collage (9) à la surface du matériau d'obturation (10) puis en déposant un second substrat (11) sur ladite résine de collage (9).

14. Procédé selon l'une quelconque des revendications 1 à 12 **caractérisé par le fait qu'**il comporte encore une étape, préalable à l'étape de dissolution de la couche de résine (5), au cours de laquelle on transfère la structure (S) de capsule nanométrique (4) sur un second substrat (11) en déposant ledit second substrat (11) à la surface du matériau d'obturation (10), ce dernier constituant une résine de collage pour le transfert de ladite structure (S) sur un second substrat (11).

15. Procédé selon l'une des revendications 13 ou 14 **caractérisé par le fait que**, suite à l'étape de transfert de la structure (S) de capsule nanométrique (4) sur un second substrat (11), l'on effectue une pulvérisation cathodique sous vide d'au moins une seconde couche (22) d'un matériau métallique ou semi-conducteur ou piézoélectrique ou isolant sur la couche de matériau encapsulant (8).

16. Procédé selon l'une quelconque des revendications 1 à 12 **caractérisé par le fait que**, suite à l'obtention d'une structure (S) de capsule nanométrique (4) par obturation de la/des cavité(s) (7) avec un matériau d'obturation (10), ledit procédé comporte encore les étapes suivantes :
- on structure, par alignement avec la première structuration de la couche de résine (5), la couche de matériau d'obturation (10) par lithographie électronique ou par lithographie optique ou par nanoimpression, de manière à éliminer ledit matériau (10) excepté au niveau de la/des capsule(s) nanométrique(s) (4) qui restent obturées ;
- on élimine la couche de matériau encapsulant (8) par gravure chimique ou par trempage ou par gravure ionique réactive ou par gravure par faisceau d'ions;
- après dissolution de la couche de résine (5) par trempage dans un solvant adapté, on libère ladite/lesdites capsule(s) nanométrique(s) (4) en solution.

## Patentansprüche

1. Verfahren zur Herstellung von mindestens einer Struktur (S) einer Nanokapsel (4), die mindestens eine Kapsel (4) nanometrischer Größe umfasst, die geeignet ist, die Einkapselung einer Verbindung zu ermöglichen, wobei das besagte Verfahren **dadurch gekennzeichnet ist, dass** es die folgenden Schritte umfasst :
- mindestens eine Harzschicht (5) wird auf einer Substratschicht (6) abgeschieden ;
- die Harzschicht (5) wird durch Niederspannung-Elektronen-Lithographie oder durch optische Lithographie oder Nanoprint-Lithographie so strukturiert, dass mindestens ein Hohlraum (7) in der Dicke der besagten Harzschicht (5) erhalten wird, wobei der besagte Hohlraum (7) eine Tiefe p aufweist, die kleiner ist als die Dicke E der Harzschicht (5) ;
- es wird eine isotrope Abscheidung von mindestens einer Schicht von Einkapselungsmaterial (8) durch Vakuum-Kathodenzerstäubung des bsagten Materials (8) durchgeführt ;
- der bzw. die Hohlräume (7) werden mit einem Dichtungsmaterial (10) verschlossen ;
- die Harzschicht (5) wird durch Eintauchen in ein geeignetes Lösungsmittel aufgelöst.

2. Verfahren nach Anspruch 1, **dadurch gekennzeichnet, dass** nach dem Schritt der isotropen Abscheidung mindestens einer Schicht von Einkapselungsmaterial (8) durch Vakuum-Kathodenzerstäubung der Hohlraum bzw. die Hohlräume (7) mit einer beliebigen Verbindung (14) gefüllt werden, wobei die Verbindung (14) flüssig, fest oder gasförmig, einschließlich Vakuum, sein kann.

3. Verfahren nach irgendeinem der vorhergehenden Ansprüche, **dadurch gekennzeichnet, dass** die Harzschicht (5) durch Elektronen-Lithographie bei Niederspannung strukturiert wird, wobei die letztere zwischen 1 und 15 kV liegt.

4. Verfahren nach Anspruch 3, **dadurch gekennzeichnet, dass** vor der Strukturierung der Harzschicht (5) durch Elektronen-Lithographie ein iterativer Annäherungstest durchgeführt wird, um die auf die Harzschicht (5) zu sendende Elektronendosis zu bestimmen, sodass ein Durchdringen der Harzschicht (5) bis an der Substratschicht (6) vermieden wird.

5. Verfahren nach irgendeinem der vorhergehenden Ansprüche, **dadurch gekennzeichnet, dass** auf der Substratschicht (6) durch Spinnbeschichtung eine Harzschicht (5) abgeschieden wird, die eine Dicke E zwischen 300 nm und 2µm aufweist, und dass die Harzschicht (5) so strukturiert wird, dass ein Hohlraum (7) erhalten wird, der eine Tiefe p zwischen 20nm und 1µm, vorzugsweise zwischen 50 und 500nm, vorzugsweise zwischen 50 und 300nm aufweist, wobei die besagte Tiefe p geringer als die Dicke E ist.

6. Verfahren nach irgendeinem der vorhergehenden Ansprüche, **dadurch gekennzeichnet, dass** die isotrope Abscheidung durch Vakuum-Kathodenzerstäubung des Einkapselungsmaterials (8) bei einer Leistung zwischen 80 und 120 Watt, vorzugsweise gleich 100 W, durchgeführt wird.

7. Verfahren nach irgendeinem der vorhergehenden Ansprüche, **dadurch gekennzeichnet, dass** alle Schritte des besagten Verfahrens bei einer kontrollierten Temperatur zwischen 15°C und 30°C, vorzugsweise zwischen 18 und 25°C, vorzugsweise gleich 20°C durchgeführt werden.

8. Verfahren nach irgendeinem der vorhergehenden Ansprüche, **dadurch gekennzeichnet, dass** mindestens eine Schicht von Einkapselungsmaterial (8), deren Dicke e zwischen 10 und 200nm liegt, gesprüht wird.

9. Verfahren nach irgendeinem der vorhergehenden Ansprüche, **dadurch gekennzeichnet, dass** eine aus Polymethylmethacrylat und/oder Polymethylmethacrylat-Methacrylsäure bestehende Harzschicht (5) abgeschieden wird.

10. Verfahren nach irgendeinem der vorhergehenden Ansprüche, **dadurch gekennzeichnet, dass** das Substrat (6) aus einem weichen oder starren aus Silizium, Zinkoxid, Aluminiumnitrid, Siliziumoxid, Aluminiumoxid, Polymeren, Kunststoffen gewählten Material besteht.

11. Verfahren nach irgendeinem der vorhergehenden Ansprüche, **dadurch gekennzeichnet, dass** ein aus Metallmaterialien, Halbleitermaterialien, piezoelektrischen Materialien und Isoliermaterialien ausgewähltes sprühbares Einkapselungsmaterial (8) abgeschieden wird.

12. Verfahren nach irgendeinem der vorhergehenden Ansprüche, **dadurch gekennzeichnet, dass** der bzw. die Hohlräume (7) mit einem aus Polymeren, Metallen, Epoxyharzen und biokompatiblen Harzen ausgewählten Dichtungsmaterial (10) verschlossen wird, wobei das Dichtungsmaterial (10) geeignet ist, dem im Schritt des Auflösens des Harzes (5) verwendeten Lösungsmittel zu widerstehen.

13. Verfahren nach irgendeinem der vorhergehenden Ansprüche, **dadurch gekennzeichnet, dass** es vor dem Schritt des Auflösens der Harzschicht (5) auch einen Schritt umfasst, während dem die Struktur (S) der Nanokapsel (4) auf ein zweites Substrat (11) übertragen wird, indem ein Leimharz (9) auf die Oberfläche des Dichtungsmaterials (10) aufgebracht wird, und anschließend ein zweites Substrat (11) auf dem besagten Leimharz (9) abgeschieden wird.

14. Verfahren nach irgendeinem der Ansprüche 1 bis 12, **dadurch gekennzeichnet, dass** es vor dem Schritt des Auflösens der Harzschicht (5) auch einen Schritt umfasst, während dem die Struktur der Nanokapsel (4) auf ein zweites Substrat (11) übertragen wird (S), indem das besagte zweite Substrat (11) auf der Oberfläche des Dichtungsmaterials (10) abgeschieden wird, wobei das letztere ein Leimharz zum Übertragen der besagten Struktur (S) auf ein zweites Substrat (11) bildet.

15. Verfahren nach einem der Ansprüche 13 oder 14, **dadurch gekennzeichnet, dass** im Anschluss an den Schritt des Übertragens der Struktur (S) der Nanokapsel (4) auf ein zweites Substrat (11) eine Vakuum-Kathoden-Zerstäubung mindestens einer zweiten Schicht (22) von metallischem oder Halbleiter- oder piezoelektrischem oder isolierendem Material auf der Schicht von Einkapselungsmaterial (8) durchgeführt wird.

16. Verfahren nach irgendeinem der Ansprüche 1 bis 12, **dadurch gekennzeichnet, dass** nach der Herstellung einer Struktur (S) einer Nanokapsel (4) durch Verschließen des bzw. der Hohlräume (7) mit einem Dichtungsmaterial (10) das Verfahren auch die folgenden Schritte umfasst :
- die Schicht aus Dichtungsmaterial (10) wird durch Ausrichten auf die erste Strukturierung der Harzschicht (5) durch elektronische Lithographie oder durch optische Lithographie oder durch Nanoimprint-Lithographie so strukturiert, dass das Material (10), außer im Bereich der verschlossen bleibenden Nanokapsel bzw. Nanokapseln (4), entfernt wird ;
- die Schicht von Einkapselungsmaterial (8) wird durch chemisches Ätzen oder durch Eintauchen oder durch reaktives Ionenätzen oder durch Ionenstrahlätzen entfernt;
- nach dem Auflösen der Harzschicht (5) durch Eintauchen in ein geeignetes Lösungsmittel werden die genannten Nanokapsel bzw. -kapseln (4) in Lösung freigegeben.

## Claims

1. A method for obtaining at least one structure (S) of a nano-capsule (4) including at least one nanometer-sized capsule (4) capable of permitting the encapsulation of a compound, wherein said method includes the following steps :
- at least one layer of resin (5) is deposited on a substrate layer (6);
- said layer of resin (5) is structured by low-voltage electronic lithography or by optical lithography or nanoimprint lithography, so as to obtain at least one cavity (7) in the thickness of said layer of resin (5), said cavity (7) having a depth p smaller than the thickness E of said layer of resin (5) ;
- an isotropic deposition of at least one layer of an encapsulating material (8) is performed by cathode sputtering under vacuum of said material (8) ;
- the cavity or cavities (7) are sealed with a sealing material (10) ;
- the layer of resin (5) is dissolved by dipping in a suitable solvent.

2. The method according to claim 1, wherein, after the step of isotropic deposition of at least one layer of encapsulating material (8) by cathode sputtering under vacuum, the cavity or cavities (7) are filled with a compound of any kind (14), whereby said compound (14) may be liquid, solid or gaseous, including vacuum.

3. The method according to any one of the preceding claims, wherein the layer of resin (5) is structured by electronic lithography at low voltage, the latter ranging between 1 and 15kV.

4. The method according to claim 3, wherein, prior to the structuring of the layer of resin (5) by electronic lithography, an iterative approach test is performed in order to determine the dose of electrons to be sent onto the layer of resin (5), so as to avoid piercing said layer of resin (5) down to the layer of substrate (6).

5. The method according to any one of the preceding claims, wherein on the layer of substrate (6) is deposited, by dip spinning, a layer of resin (5) having a thickness E ranging between 300 nm and 2 µm and said layer of resin (5) is structured so as to obtain a cavity (7) having a depth p ranging between 20 nm and 1 µm, preferably between 50 and 500 nm, preferably between 50 and 300 nm, said depth p being smaller than said thickness E.

6. The method according to any one of the preceding claims, wherein the isotropic deposition is performed by cathode sputtering under vacuum encapsulating material (8) at a power between 80 and 120 Watts, preferably equal to 100W.

7. The method according to any one of the preceding claims, wherein all the steps of said method are performed at a controlled temperature ranging between 15°C and 30°C, preferably between 18 and 25°C, preferably equal to 20°C.

8. The method according to any one of the preceding claims, wherein at least one layer of encapsulating material (8) is sprayed, the thickness e of which ranges between 10 and 200 nm.

9. The method according to any one of the preceding claims, wherein a layer of resin (5) consisting of (poly)methyl-methacrylate and/or poly(methyl-methacrylate methacrylic acid) is deposited.

10. The method according to any one of the preceding claims, wherein the substrate (6) is formed of a flexible or rigid material chosen from silicon, zinc oxide, aluminum nitride, silicon oxide, aluminum oxide, polymers, plastic materials.

11. The method according to any one of the preceding claims, wherein a sprayable encapsulating material (8) chosen from metal materials, semiconductor materials, piezoelectric materials and insulating materials is deposited.

12. The method according to any one of the preceding claims, wherein the cavity or cavities (7) are sealed with a sealing material (10) chosen from polymers, metals, epoxy resins and biocompatible resins, said sealing material (10) being capable of withstanding the solvent used in the step of dissolving the resin (5).

13. The method according to any one of the preceding claims, wherein it also comprises a step, prior to the step of dissolving the layer of resin (5), during which the structure (S) of the nano-capsule (4) is transferred onto a second substrate (11) by applying a bonding resin (9) on the surface of the sealing material (10) and then depositing a second substrate (11) on said bonding resin (9).

14. The method according to any one of claims 1 to 12, wherein it also comprises a step, prior to the step of dissolving the layer of resin (5), during which the structure (S) of the nano-capsule (4) is transferred onto a second substrate (11) by depositing said second substrate (11) on the surface of the sealing material (10), the latter forming a bonding resin for the transfer of said structure (S) onto a second substrate (11).

15. The method according to one of claims 13 or 14, wherein after the step of transferring the structure (S) of the nano-capsule (4) onto a second substrate (11) is performed a cathode sputtering under vacuum of at least a second layer (22) of a metallic or semiconductor or piezoelectric or insulating material onto the layer of encapsulating material (8).

16. The method according to any one of claims 1 to 12, wherein, after obtaining a structure (S) of the nano-capsule (4) by sealing the cavity or cavities (7) with a sealing material (10), said method also includes the following steps :
- the layer of sealing material (10) is structured through alignment with the first structuring of the layer of resin (5) by electronic lithography or by optical lithography or nanoimprint lithography, so as to remove said material (10) except at the level of the nano-capsule or capsules (4), which remain sealed ;
- the layer of encapsulating material (8) is removed by chemical etching or by dipping or by reactive ion etching or by ion beam etching ;
- after dissolving the layer of resin (5) by dipping in a suitable solvent, said nano-capsule or capsules (4) are released in a solution.
